# EUROPÄISCHE PATENTANMELDUNG

(11) **EP 1 238 956 A1**
(43) Veröffentlichungstag der Anmeldung: **11.09.2002**
(21) Anmeldenummer: 01105991.2
(22) Anmeldetag: 10.03.2001
(51) Int. Cl.: C04B 38/00, C04B 38/06, C04B 41/83, A61K 6/06, A61K 6/083

(54) **Verbundwerkstoff und Verfahren zu seiner Herstellung**

(71) Anmelder: Vita Zahnfabrik H. Rauter GmbH & Co. KG, D-79704 Bad Säckingen (DE)
(72) Erfinder: Aechtner, Stefan, 79713 Bad Säckingen (DE); Hornberger, Helga, 79730 Murg-Hänner (DE); Nagel, Emil, 79713 Bad Säckingen (DE); Thiel, Norbert, 79713 Bad Säckingen (DE)
(74) Vertreter: Meyers, Hans-Wilhelm, Dr.Dipl.-Chem.

(57) **Zusammenfassung**

Verbundwerkstoff mit einer porösen anorganisch nichtmetallischen Matrix und einem zweiten Material, dadurch gekennzeichnet, dass
- die poröse anorganische nichtmetallische Matrix eine Biegebruchfestigkeit von ≥ 40 Mpa, gemessen nach ISO 6 872, besitzt,
- das zweite Material ein organisches Material ist, das die Poren der porösen Matrix mindestens teilweise ausfüllt und
der Verbundwerkstoff einen Elastizitätsmodul E ≥ 25 GPa gemessen nach ISO 10 477 aufweist.

## Beschreibung

Die vorliegende Erfindung betrifft einen Verbundwerkstoff mit einem porösen anorganisch-nichtmetallischen Netzwerk, dessen Poren mit einem Polymer gefüllt sind, ein Verfahren zu seiner Herstellung, sowie Verwendungen dieses Verbundwerkstoffs.

Die EP-A-1 006 095 betrifft ein Verfahren zur Herstellung endodontischer, orthodontischer und direkter Restaurationen aufbauend auf einem keramischen Netzwerk. Ausgehend von einem Glasmonomer, wird dieses mit einem Material gefüllt, um verbesserte Flexibilität und Abriebbeständigkeit zu erreichen. Das Infiltrationsmaterial kann beispielsweise ein Glas sein, das dann eine Schicht bildet mit einer Härte im Bereich von 300 - 600 KHN und einem Elastizitätsmodul im Bereich von 70 - 80 GPa aufweist. Die Biegebruchfestigkeit liegt im Bereich von 200 - 500 MPa. Im Inneren soll die Struktur eine Biegebruchfestigkeit von etwa 150 - 80 MPa und einen Elastizitätsmodul von 15 - 25 GPa aufweisen. Entsprechende Werte sollen durch Infiltration mit einem Monomer und anschließender Härtung erreicht werden. Damit ist ein zweischichtiger Aufbau beschrieben, der unterschiedliche Eigenschaften aufweist. Entsprechende Produkte sind bislang nicht bekannt.

Die EP-A-0 803 241 betrifft ein Dentalmaterial aus einer porösen Keramik, die in einen künstlichen Zahn gefüllt wird, ein Inlay, ein Onlay, eine Krone, eine Kronenbrücke oder in einem Block, der geeignet für CAD/CAM-Verarbeitung ist. Dieses Material wird gesintert, wobei miteinander in Verbindung stehende Poren entstehen. Anschließend wird mit einem Kunstharz imprägniert. Nachteilig an diesen beschriebenen Produkten ist die nicht genaue Definition und Beschreibung des zugrundeliegenden Netzwerks. Dies führt in vielen Ausführungen zur Verschlechterung der physikalisch-technischen Daten im Vergleich zu heute gängigen Kompositmaterialien. Die US-A-5,843,348 betrifft ein Verfahren zur Herstellung eines keramischen Netzwerkmaterials aus einer keramischen Suspension. Dieses Verfahren beinhaltet das Gießen einer Suspension in eine Form, die zahnförmig ausgestaltet ist. Die Suspension enthält dispergierte Aluminiumoxidpartikel in einem Medium, das deionisiertes Wasser mit einem pH im Bereich von 4 - 5 und Polyvinylalkohol in einer Konzentration von 0,5 - 1 Gew.-% enthält. Nach Trocknung der Suspension wird diese in einem Ofen bei einer Temperatur im Bereich von 1000 - 1400°C gebrannt, um ein keramisches Netzwerk zu erzeugen. Die poröse Struktur wird mit Lanthanaluminosilikatglas infundiert, so dass eine Glasschicht mit einer Dicke in einem Bereich von 1 - 2 mm innerhalb des keramischen Netzwerkes entsteht.

In Ullmanns Encyclopedia of Industrial Chemistry werden verschiedene Verfahren zur Konservierung von porösen Steinstrukturen beschrieben. Dabei werden die Objekte, z.B. Skulpturen aus Sandstein oder Marmor, mit hydrophoben Reagenzien wie Alkyltrialkoxysilanen und/oder Kopplungsreagenzien wie Tetraethyorthosilikat imprägniert. Zur Stabilisierung der Struktur werden die Poren außerdem unter Vakuum mit Monomeren, beispielsweise Methylmethacrylat, infiltriert. Nach der thermischen Aushärtung unter Druck sind die Objekte mechanisch stabiler und vor allem ist dem chemischen Zerfall durch den Verschluss der Poren weitgehend Einhalt geboten.

Der Erfindung liegt die Aufgabe zu Grunde, einen Verbundwerkstoff zu schaffen, der ein homogenes porenfreies Gefüge und Eigenschaften aufweist, die zwischen keramischen und konventionell gefüllten Polymerwerkstoffen liegen, und ein Verfahren zur Herstellung dieses Verbundwerkstoffs aufzuzeigen.

Diese Aufgabe wird gelöst durch einen Verbundwerkstoff mit einer porösen anorganisch nichtmetallischen Matrix und einem zweiten Material, dadurch gekennzeichnet, dass
- die poröse anorganische nichtmetallische Matrix eine Biegebruchfestigkeit von ≥ 40 Mpa, gemessen nach ISO 6 872, besitzt,
- das zweite Material ein organisches Material ist, das die Poren der porösen Matrix mindestens teilweise ausfüllt und
der Verbundwerkstoff einen Elastizitätsmodul E ≥ 25 GPa gemessen nach ISO 10 477 aufweist.

Der erfindungsgemäße Verbundwerkstoff ist vorteilhaft, weil eine neue Werkstoffklasse erreicht wird, deren Eigenschaften zwischen Keramik- und Kunststoffmaterialien liegen. Zum Beispiel zeichnet sich diese Werkstoffklasse einerseits durch eine geringere Sprödigkeit als Keramik und andererseits durch eine erhöhte Abriebfestigkeit gegenüber den bisherigen anorganisch gefüllten Polymeren aus.

Die anorganisch-nichtmetallische Phase des erfindungsgemäßen Verbundwerkstoffs bildet ein Netzwerk, das eine Eigenfestigkeit (Biegebruchfestigkeit des porösen anorganisch-nichtmetallischen Netzwerks vor Infiltration) von σ ≥ 40 MPa besitzt. Dadurch findet eine Erhöhung des Elastizitätsmodul des Verbundwerkstoffes im Vergleich zu herkömmlich gefüllten Kompositen statt.

Der erfindungsgemäße Verbundwerkstoff weist vorzugsweise eine Biegebruchfestigkeit von σ ≥ 100 Mpa, gemessen nach ISO 6 872 auf.

Die Poren des porösen Netzwerks nehmen ein Volumen von 5 % bis 85 Vol%, vorzugsweise 10 bis 50 Vol%, insbesondere 15 bis 35 Vol% ein. Ein Vorteil einer einstellbaren Porosität des anorganisch-nichtmetallischen Netzwerks ist der damit korrelierende Polymeranteil im späteren Verbundwerkstoff. Die Erhöhung des Polymeranteils ist verbunden mit einer Erhöhung der Schlagzähigkeit und der Abnahme des spezifischen Gewichts des Verbundwerkstoffs.

Typischerweise weisen die Poren des anorganisch-nichtmetallischen Netzwerks des erfindungsgemäßen Verbundwerkstoffs eine Porengröße von 0,2 bis 25 µm, vorzugsweise 0,5 bis 10 µm, auf. Die zu verwendenden Porengrößen und -formen sind abhängig von der Werkstoffkombination aus Anorganik und Polymer, vom Benetzungswinkel zwischen Anorganik und Monomer bzw. Polymer, von der Vorbehandlung und vom verwendeten Infiltrationsverfahren.

Das poröse anorganisch-nichtmetallische Netzwerk als Zwischenschritt zum erfindungsgemäßen Verbundwerkstoff ist beispielsweise durch Sinterung von pulverförmigen anorganischen, insbesondere keramischen Substanzen oder Substanzmischungen erhältlich, wobei der Sinterungsprozess insbesondere vor der Bildung geschlossener Poren beendet wird. Bevorzugt wird weiterhin, dass der Sinterprozess erst nach der Phase der Sinterhalsbildung oder des Glasflusses beendet wird, die typischerweise mit einer sprunghaften Änderung der Eigenfestigkeit des anorganisch-nichtmetallischen Netzwerkes verbunden ist.

Vorzugsweise werden pulverförmige anorganische Substanzen oder Substanzmischungen mit Korngrößen eingesetzt, die eine bimodale Verteilung, z.B. feine und grobe Korngrößen, aufweisen. Dabei weisen die feinen Körner eine höhere Sinteraktivität auf, die großen Körner bestimmen die Porenform.

Das anorganisch-nichtmetallische Netzwerk des erfindungsgemäßen Verbundwerkstoffs weist in einer bevorzugten Ausführungsform pulverförmige anorganische Substanzen oder Substanzmischungen auf, die eine Korngröße von 0,2. µm bis 25 um besitzen. Typische d₅₀ Werte (Lasergranulometrie) der verwendeten Ausgangsmaterialien liegen zwischen 0,5 und 5 µm.

Das anorganisch-nichtmetallische Netzwerk des erfindungsgemäßen Verbundwerkstoffs ist vorzugsweise aus mindestens zwei verschiedenen Pulvermischungen mit unterschiedlichen Sintertemperaturen aufgebaut worden. Die Sinteraktivität wird durch die niedrigschmelzenden Pulverkomponenten bestimmt.

Die Poren des anorganisch-nichtmetallischen Netzwerks weisen vorteilhafterweise Oberflächen auf, die hydrophobe Eigenschaften besitzen. Die hydrophopen Eigenschaften können zum Beispiel durch oberflächliche Silanisierung erreicht werden. Die hydrophoben Oberflächen erhöhen die Benetzbarkeit des porösen Netzwerks mit Monomeren.

Die Silanisierung erfolgt in einfacher Weise mittels eines Silanisierungmittels in flüssiger Phase.

Zur Silanisierung des porösen anorganisch-nichtmetallischen Netzwerks wird ein Alkoxysilan oder ein Halogensilan, vorzugsweise 3-Methacryloxypropyl-trimethoxysilan, eingesetzt.

Die organische Phase des erfindungsgemäßen Verbundwerkstoffs ist insbesondere ein organisches Polymer, das durch Präpolymere, Oligomere oder Monomere in situ in den Poren des porösen Netzwerks durch Polymerisation gebildet wird. Das organische Polymer wird aus thermisch polymerisierbaren Monomeren und/oder durch chemisch induzierte Starterreaktionen polymerisierbarer Monomere und/oder durch kondensierbare Monomere gebildet.

Der erfindungsgemäße Verbundwerkstoff ist vorzugsweise isotrop. Dadurch entfallen die Nachteile der heutigen anisotropen Komposite, die z.B. bei Beanspruchung gegen die Vorzugsrichtung nur geringe Festigkeiten aufweisen. Dennoch ist es vorstellbar, dass eine gezielte Anisotropie in den erfindungsgemäßen Verbundwerkstoff eingebracht werden kann. Dies kann z.B. durch Einbringen von Fasern, die in andere Raumrichtungen gezielt vernetzt werden, erfolgen. Des weiteren ist durch Verwendung von Gradientenöfen zur Herstellung des anorganisch-nichtmetallischen Netzwerks, die Herstellung von kontinuierlichen Gradientenwerkstoffen möglich.

Der erfindungsgemäße Verbundwerkstoff kann Hilfsstoffe, wie Antioxidantien und für den jeweiligen Anwendungszweck geeignete Pigmente aufweisen.

Der erfindungsgemäße Verbundwerkstoff kann z. B. durch ein Verfahren wie folgt hergestellt werden:
- Herstellen des anorganisch-nichtmetallischen Ausgangsmaterials,
- Formgebungsverfahren der anorganisch-nichtmetallischen Phase, nass z.B. Schlickern oder trocken z. B. isostatisches Pressen, gegebenenfalls unter Verwendung eines geeigneten Bindersystems.
- Sintern des anorganisch-nichtmetallischen Netzwerks auf den gewünschten Sintergrad und Porosität,
- wobei der Sinterprozess beendet wird, bevor im wesentlichen geschlossene Poren im Sinterprodukt entstehen,
- und nachdem die Phase der Sinterhalsbildung und/oder Glasflusses erreicht worden ist,
- das erhaltene poröse Produkt aus anorganisch-nichtmetallischen Material wird zunächst mit einem Benetzungsmittel, bevorzugt Silane mit einer geeigneten funktionellen Gruppe, belegt,
- dann wird das anorganisch-nichtmetallische Netzwerk vollständig mit Monomeren infiltriert,
- und anschließend mit einem geeigneten Verfahren, wie z.B. Heißpolymerisation oder Mikrowellen, polymerisiert.

Im erfindungsgemäßen Verfahren werden als anorganisches Material z.B. Oxidkeramiken, Gläser, Porzellane, Nichtoxidkeramiken und Kombinationen derselben eingesetzt.

Im erfindungsgemäßen Verfahren werden insbesondere pulverförmige anorganische Materialien mit einer Korngröße von 0,2 µm bis 25 µm, vorzugsweise 0,5 bis 10 µm (d₅₀-Werte bestimmt mittels Lasergranulometrie), eingesetzt.

Vorzugsweise wird erfindungsgemäß das organische Material in flüssiger Form in das gesinterte anorganische Material infiltriert.

Das Benetzungsmittel liegt vorzugsweise in Lösung vor. Ein Vorteil der Verdünnung liegt in der erniedrigten Viskosität.

Das Benetzungsmittel muss eine kopplungsfähige funktionelle Gruppe enthalten.

Erfindungsgemäß werden organische Monomere oder Präpolymere in das gesinterte anorganisch-nichtmetallische Netzwerk eingebracht und in den Poren des Netzwerks zu dem organischen Material polymerisiert.

Das organische Material kann erfindungsgemäß durch Druckinfiltration in das gesinterte anorganische Material eingebracht werden. Der Vorteil besteht in der schnellen vollständigen und homogenen Durchdringung. Es kann sich je nach Aufgabenstellung als Vorteil erweisen, die Polymerisation unter Druck durchzuführen.

Gegebenenfalls sind vor der Infiltration sowohl das anorganisch-nichtmetallische Netzwerk als auch das organische Monomer zu evakuieren.

Als Monomere werden dabei vorzugsweise organische Verbindungen eingesetzt, die mindestens eine ethylenisch ungesättigte Struktureinheit, mindestens eine kondensierbare Struktureinheit oder mindestens eine ringöffnende polymerisierbare Struktureinheit oder Kombinationen derselben aufweisen.

Geeignete Startersysteme für die Polymerisation sind bekannt und der entsprechenden Literatur zu entnehmen.

Zur Herstellung transluzenter für Dentalzwecke geeigneter Materialien werden erfindungsgemäß feldspathaltige Pulver und Fritten als anorganisches Material und Bismethacrylate als organische Verbindung sowie peroxidhaltige Verbindungen als Starter eingesetzt.

Gegenstand der Erfindung ist auch ein Verbundwerkstoff für dentale Anwendungen, der durch das erfindungsgemäße Verfahren erhältlich ist.

Der erfindungsgemäße Verbundwerkstoff kann bevorzugt für dentale Zwecke, wie z.B. für Inlays, Onlays, Kronen und Brücken verwendet werden.

Erfindungsgemäß hergestellte Verbundwerkstoffe sind u.a. verwendbar zur Oberflächenvergütung von keramischen und metallischen Werkstoffen, Kompositen und Kunststoffen, als Bauteile mit neuartigen Eigenschaften wie z.B. Elastizitätsmodul, Abrasionsverhalten, spez. Gewicht, Formstabilität bei erhöhten Temperaturen. Weitere Ausführungen sind schalldämmende und wärmedämmende Elemente, Gleitlager, Elemente zur Vibrationsdämpfung, elektrische Isolatoren usw.

Insbesondere kann der erfindungsgemäße Verbundwerkstoff als Gleitlager, zur Wärme- und/oder Schalldämmung, oder als Vibrationsdämpfer eingesetzt werden.

Die zur Herstellung des erfindungsgemäßen Verbundwerkstoffes einsetzbare poröse anorganische nichtmetallische Matrix mit einer Biegebruchfestigkeit von ≥ 40 Mpa, gemessen nach ISO 6 872 kann als Zwischenprodukt dienen und ist ebenfalls Gegenstand der Erfindung.

Vorzugsweise wird die erfindungsgemäße poröse anorganische nichtmetallische Matrix aus Oxidkeramik, Gläsern, Porzellanen, Nichtoxidkeramiken oder Kombinationen davon gebildet.

Die Poren der erfindungsgemäßen porösen anorganischen nichtmetallischen Matrix nehmen insbesondere ein Volumen von 5 Vol % bis 85 Vol %, vorzugsweise 10 bis 50 Vol %, insbesondere 15 - 35 Vol % ein.

Erfindungsgemäß beansprucht wird auch ein Verfahren zur Herstellung einer erfindungsgemäßen porösen anorganisch-nichtmetallischen Matrix,
- wobei ein anorganisches nichtmetallisches Material mit einem entfernbaren Binder zu einem formbaren Material gemischt wird,
- der Binder entfernt wird unter Erhalt einer porösen anorganischen nichtmetallischen Struktur,
- die poröse anorganische nichtmetallische Struktur gesintert wird,
- unter Ausbildung der porösen anorganisch- nichtmetallischen Matrix.

Ein Verfahren zur Abformung von Gegenständen, unter Verwendung der erfindungsgemäßen porösen anorganisch-nichtmetallischen Matrix ist ebenfalls Gegenstand der Erfindung. Dieses erfindungsgemäße Verfahren ist dadurch gekennzeichnet, dass
- ein anorganisches nichtmetallisches Material mit einem entfernbaren Binder zu einem formbaren Material gemischt wird,
- das formbare Material mit einem abzuformenden Gegenstand in Kontakt gebracht wird, so dass der abzuformende Gegenstand in Negativform im formbaren Material abgeformt wird,
- das formbare Material unter Erhalt der Form des abgeformten Gegenstandes abgelöst wird und dann der Binder entfernt wird,
- danach gegebenenfalls die nach dem Entfernen des Binders erhaltene Struktur gesintert und infiltriert wird.

In einer Weiterbildung des genannten Verfahrens wird ein Verfahren zur Replikation von Gegenständen erhalten. Dabei wird zunächst das erfindungsgemäße Verfahren zur Abformung von Gegenständen durchgeführt. Die dadurch erhaltene Matrix wird selbst abgeformt, unter Erhalt eines Replikates des zu replizierenden Gegenstands. Es kann aber auch von einer auf andere Weise gewonnenen Abformung eines Gegenstandes ausgegangen werden, unter Erhalt einer Negativform, die dann gemäß des erfindungsgemäßen Abformverfahrens abgeformt und gegebenenfalls verfestigt wird.

Erfindungsgemäß beansprucht wird auch eine Mischung aus pulverförmigen anorganischen nichtmetallischen Substanzen oder Substanzmischungen und entfernbarem Binder. Diese Mischung ist dadurch gekennzeichnet, dass die pulverförmigen anorganischen nichtmetallischen Substanzen oder Substanzmischungen eine Korngröße d₅₀ von 0,2 µm bis 25 µm aufweisen und der Binder in einer Menge von 2 Gew % bis 50 Gew %, bezogen auf das Gesamtgewicht der Mischung, vorliegt.

Die pulverförmigen anorganischen nichtmetallischen Substanzen oder Substanzmischungen sind vorzugsweise Oxidkeramik, Gläser, Porzellane, Nichtoxidkeramiken oder Kombinationen davon.

Vorzugsweise werden pulverförmige anorganische Substanzen oder Substanzmischungen mit Korngrößen eingesetzt, die eine bimodale Verteilung aufweisen.

Die pulverförmigen anorganischen nichtmetallischen Substanzen oder Substanzmischungen zur Herstellung der erfindungsgemäßen Paste weisen insbesondere eine Korngröße d₅₀ von 0,5 bis 5 µm, gemessen mittels Lasergranulometrie, auf.

In der erfindungsgemäßen Mischung zur Herstellung der Paste können mindestens zwei verschiedene Pulver und/oder Pulvermischungen mit unterschiedlichen Sintertemperaturen vorliegen. In der erfindungsgemäßen Paste sind Zusätze enthalten, die ein gezieltes Aushärten nach Stand der Technik erlauben.

Mittels der erfindungsgemäßen porösen anorganisch nichtmetallischen Matrix lassen sich Formkörper erhalten, die vorteilhafte Eigenschaften besitzen und auch Gegenstand der Erfindung sind. Die erfindungsgemäßen Formkörper sind aus porös gesinterten natürlichen und/oder synthetischen Feldspäten oder Feldspatoiden mit einer Porosität entsprechend einem Volumen von 5 Vol % bis 85 Vol %, vorzugsweise 10 bis 50 Vol %, insbesondere 15 - 35 Vol % herstellbar. Durch Infiltration mit geeigneten Monomeren und anschließender Polymerisation sind transparente, für dentale Anwendung (z.B. Verwendung als Inlays, Onlays, Veneers Kronen oder Brücken) geeignete Werkstoffe herstellbar.

Insbesondere vorteilhaft sind mehrschichtige Formkörper mit mehreren Schichten mit unterschiedlichen Eigenschaften in den Schichten erhältlich durch Auftragen verschiedener Pasten mit unterschiedlichen resultierenden anorganisch-nichtmetallischen Matrixkomponenten, anschließendem Sintern D Die Herstellung des kompletten Verbundwerkstoffs erfolgt dann wie bereits beschrieben.

Auch Formkörper mit kontinuierlich ändernden Eigenschaften sind gemäß der Erfindung herstellbar und Gegenstand der Erfindung. Diese Formkörper sind erhältlich durch z.B. Sintern der anorganisch-nichtmetallischen Matrix in einem Gradientenofen. Auch der natürliche Zahn ist anisotrop und aus mehreren unterschiedlichen Schichten aufgebaut Typischerweise werden die erfindungsgemäßen Formkörper wie folgt hergestellt
- Präparation der Arbeit im Mund, Aufbringen eines Trennmittels
- bei Kavitäten direkte Abformung im Mund mittels der erfindungsgemäßen Paste
- Härtung oder Aushärtung des Formkörpers, Entfernen aus der Kavität
- Sintern auf Porosität unter Ausbrennen des Binders.

- bei Kronen und Brücken: Abformung der Präparation
- Erstellen des Meistermodells
- Aufbau der Restauration auf dem Modell mittels erfindungsgemäßer Paste
- Sintern der Arbeit auf Porosität unter Ausbrennen des Binders.

Die Erfindung wird anhand der folgenden Beispiele näher erläutert.

### Beispiel 1:

Ein bimodales Aluminiumoxid mit einem d-50 von ca. 2,5 µm wurde mit destilliertem Wasser und üblichen Zusätzen (hier Citronensäure und Darvan) sowie Ultraschall zu einer schlicker-fähigen Suspension angerührt. Mit dieser Suspension wurden Delrinformen mit den Maßen 1,2x4x20mm ausgegossen. Nach Trocknung wurden die Teile entformt und bei einer Spitzentemperatur von 1120 °C und einer Haltezeit von 2 Stunden gebrannt.

Die gebrannten porösen Teile wurden dann mit einer 5%igen Lösung von Methacryloxypropyl-Trimethoxysilan getränkt und erneut getrocknet. Danach wurden die Teile evakuiert und mit einer evakuierten 1:1-Mischung aus Urethandimethacrylat und Triethylenglykoldimethacrylat über Nacht bei Raumtemperatur infiltriert und anschließend über einen Zeitraum von 17 Std. polymerisiert , wobei die Spitzentemperatur 80 °C betrug.

Die Teile zeigten auf einer Universalprüfmaschine der Fa. Zwick eine mittlere Biegefestigkeit von 300,15 MPa und einen Elastizitätsmodul von 76,23 GPa.

### Beispiel 2:

Ein bimodales Magnesium-Aluminiumoxid-Spinell mit einem d-50 von ca. 2,5µm wurde mit destilliertem Wasser und üblichen Zusätzen (hier Citronensäure und Darvan) sowie Ultraschall zu einer schlickerfähigen Suspension angerührt. Mit dieser Suspension wurden Delrinformen mit den Maßen 1,2x4x20mm ausgegossen. Nach Trocknung wurden die Teile entformt und bei einer Spitzentemperatur von 1180 °C und einer Haltezeit von 2 Stunden gebrannt.

Die gebrannten porösen Teile wurden dann mit einer 5%igen Lösung von Methacryloxypropyl-Trimethoxysilan getränkt und erneut getrocknet. Danach wurden die Teile evakuiert und mit einer evakuierten 1:1-Mischung aus Urethandimethacrylat und Triethylenglykoldimethacrylat über Nacht bei Raumtemperatur infiltriert und anschließend über einen Zeitraum von 17 Std. polymerisiert, wobei die Spitzentemperatur 80 °C betrug.

Die Teile zeigten auf einer Universalprüfmaschine der Fa. Zwick eine mittlere Biegefestigkeit von 256,87 MPa und einen Elastizitätsmodul von 82,89 GPa.

### Beispiel 3:

Ein bimodales Gemisch aus 67% Aluminiumoxid und 33% Zirkondioxid mit einem d-50 von ca. 2,5 µm wurde mit destilliertem Wasser und üblichen Zusätzen (hier Citronensäure und Darvan) sowie Ultraschall zu einer schlickerfähigen Suspension angerührt. Mit dieser Suspension wurden Delrinformen mit den Maßen 1,2x4x20mm ausgegossen. Nach Trocknung wurden die Teile entformt und bei einer Spitzentemperatur von 1180 °C und einer Haltezeit von 2 Stunden gebrannt.

Die gebrannten porösen Teile wurden dann mit einer 5%igen Lösung von Methacryloxypropyl-Trimethoxysilan getränkt und erneut getrocknet. Danach wurden die Teile evakuiert und mit einer evakuierten 1:1-Mischung aus Urethandimethacrylat und Triethylenglykoldimethacrylat über Nacht bei Raumtemperatur infiltriert und anschließend über einen Zeitraum von 17 Std. polymerisiert, wobei die Spitzentemperatur 80 °C betrug.

Die Teile zeigten auf einer Universalprüfmaschine der Fa. Zwick eine mittlere Biegefestigkeit von 287,42 MPa und einen Elastizitätsmodul von 79,12 GPa.

### Beispiel 4:

Ein bimodales Gemisch aus 2 Feldspatfritten (Fritte 1 Brenntemperatur ca. 830 °C, 10% Anteil an der Mischung und Fritte 2 Brenntemperatur ca. 1180 °C, 90% Anteil an der Mischung) mit einem d-50 von ca. 4,5µm wurde mit einer üblichen Modellierflüssigkeit (Wasser + Binderzusatz) zu einer schlickerfähigen Suspension angerührt. Diese Suspension wurde in Metallformen mit den Maßen 25x5x1,6 mm eingerüttelt. Nach Trocknung wurden die Teile entformt und bei einer Spitzentemperatur von 940 °C und einer Haltezeit von ca. 40 Minuten gebrannt.

Die gebrannten porösen Teile wurden dann mit einer 5%igen Lösung von Methacryloxypropyl-Trimethoxysilan getränkt und erneut getrocknet. Danach wurden die Teile evakuiert und mit einer evakuierten 1:1-Mischung aus Urethandimethacrylat und Triethylenglykoldimethacrylat über Nacht bei Raumtemperatur infiltriert und anschließend über einen Zeitraum von 17 Std. polimerisiert, wobei die Spitzentemperatur 80 °C betrug.

Die Teile zeigten auf einer Universalprüfmaschine der Fa. Zwick eine mittlere Biegefestigkeit von 148,83 MPa und einen Elastizitätsmodul von 30,04 GPa.

Die Teile zeigen eine hervorragende Transluzenz und sind auf Grund ihrer optischen Eigenschaften für ästhetische Dentalrestaurationen geeignet.

## Patentansprüche

1. Verbundwerkstoff mit einer porösen anorganisch nichtmetallischen Matrix und einem zweiten Material, **dadurch gekennzeichnet, dass**
- die poröse anorganische nichtmetallische Matrix eine Biegebruchfestigkeit von ≥ 40 Mpa, gemessen nach ISO 6 872, besitzt,
- das zweite Material ein organisches Material ist, das die Poren der porösen Matrix mindestens teilweise ausfüllt und
- der Verbundwerkstoff einen Elastizitätsmodul E ≥ 25 GPa gemessen nach ISO 10 477 aufweist.

2. Verbundwerkstoff nach Anspruch 1, **dadurch gekennzeichnet, dass** er eine Biegebruchfestigkeit von σ ≥ 100 Mpa, gemessen nach ISO 6 872 aufweist.

3. Verbundwerkstoff nach Anspruch 1 und/oder 2, **dadurch gekennzeichnet, dass** der Sinterprozess erst nach der Phase der Sinterhalsbildung und/oder des Glasflusses beendet wird.

4. Verbundwerkstoff nach mindestens einem der Ansprüche 1 bis 3, **dadurch gekennzeichnet, dass** pulverförmige anorganische Substanzen oder Substanzmischungen mit Korngrößen eingesetzt werden, die eine bimodale Verteilung aufweisen.

5. Verbundwerkstoff nach mindestens einem der Ansprüche 1 bis 4, **dadurch gekennzeichnet, dass** die pulverförmigen anorganischen Substanzen oder Substanzmischungen eine Korngröße d₅₀ von 0,2 µm bis 25 µm, vorzugsweise von 0,5 bis 5 µm, gemessen mittels Lasergranulometrie, aufweisen.

6. Verbundwerkstoff nach mindestens einem der Ansprüche 1 bis 5, **dadurch gekennzeichnet, dass** das anorganisch- nichtmetallische Netzwerk eine mit einem Kopplungsreagenz belegt Oberfläche aufweist, die dadurch hydrophobiert und mit funktionellen Gruppen versehen wird.

7. Verbundwerkstoff nach mindestens einem der Ansprüche 1 bis 6, **dadurch gekennzeichnet, dass** das Silanisierungsmittel Aminopropyltriethoxysilan, Vinyltriethoxysilan, 3-Methacryloxypropyl-trimethoxysilan oder eine Mischung derselben ist.

8. Verfahren zur Herstellung eines Verbundwerkstoffs nach mindestens einem der Ansprüche 1 bis 7, **dadurch gekennzeichnet, dass** es folgende Schritte umfasst:
- Herstellung des anorganischen Ausgangsmaterials, vorzugsweise in Pulverform,
- Sintern des anorganischen Ausgangsmaterials, wobei der Sinterprozess beendet wird, bevor im wesentlichen geschlossene Poren im Sinterprodukt entstehen,
- und die Phase der Sinterhalsbildung und/oder des Glasflusses erreicht worden ist,
- die Oberfläche der porösen anorganisch nichtmetallischen Matrix mit einem Kopplungsreagenz belegt wird,
- die so erhaltene poröse und oberflächenmodifizierte anorganische nichtmetallische Matrix mit organischem Material infiltriert wird und
- das organische Material danach verfestigt wird.

9. Verfahren nach Anspruch 8, **dadurch gekennzeichnet, dass** organische Monomere oder Präpolymere in das gesinterte anorganische Material verbracht werden und in den Poren des gesinterten anorganischen Materials zu dem organischen Material polymerisiert werden.

10. Verfahren nach Anspruch 9, **dadurch gekennzeichnet, dass** die Polymerisation unter Druck durchgeführt wird.

11. Verfahren nach Anspruch 9 und/oder 10, **dadurch gekennzeichnet, dass** als Monomere organische Verbindungen eingesetzt werden, die mindestens eine ethylenisch ungesättigte Struktureinheit, mindestens eine kondensierbare Struktureinheit und/oder mindestens eine ringöffnend polymerisierbare Struktureinheit enthalten.

12. Verfahren nach Anspruch 9 bis 11, **dadurch gekennzeichnet, dass** radikalisch polymerisierbare Monomere, wie Acrylate, Methacrylate, zusammen mit Radikalbildnern als Starter, wie Azoverbindungen oder Peroxiden, eingesetzt werden.

13. Verfahren nach mindestens einem der Ansprüche 8 bis 12, **dadurch gekennzeichnet, dass** feldspathaltige Pulver und Fritten als anorganisches Material und Bismethacrylate als organische Verbindung sowie peroxidhaltige Verbindungen als Starter eingesetzt werden.

14. Verbundwerkstoff erhältlich durch ein Verfahren nach mindestens einem der Ansprüche 8 bis 13.

15. Verwendung eines Verbundwerkstoffs nach mindestens einem der Ansprüche 1 bis 7 und/oder 14 für dentale Zwecke, z.B. für Inlays, Onlays, Kronen und Brücken.

16. Verwendung eines Verbundwerkstoffs nach mindestens einem der Ansprüche 1 bis 7 und/oder 14 als Gleitlager, als Wärme- und/oder Schalldämmung, Vibrationsdämpfer, sowie zur Oberflächenvergütung von Metallen, Kompositen und Keramiken.

17. Poröse anorganische nichtmetallische Matrix mit einer Biegebruchfestigkeit von ≥ 40 Mpa, gemessen nach ISO 6 872.

18. Verfahren zur Herstellung einer porösen anorganischen nichtmetallischen Matrix nach Anspruch 17,
- wobei ein anorganisches nichtmetallisches Material mit einem entfernbaren Binder zu einem formbaren Material gemischt wird,
- der Binder entfernt wird, unter Erhalt einer porösen anorganischen nichtmetallischen Struktur,
- die poröse anorganische nichtmetallische Struktur gesintert wird,
- unter Ausbildung der porösen anorganischen nichtmetallischen Matrix.

19. Mischung aus pulverförmigen anorganischen nichtmetallischen Substanzen oder Substanzmischungen und entfernbarem Binder, **dadurch gekennzeichnet, dass** die pulverförmige anorganische nichtmetallische Substanzen oder Substanzmischungen eine Korngröße d₅₀ von 0,2 µm bis 25 µm aufweisen und der Binder in einer Menge von 2 Gew % bis 50 Gew % bezogen auf das Gesamtgewicht der Mischung vorliegt.

20. Formkörper aus porös gesinterten natürlichen und/oder synthetischen Feldspäten oder Feldspatoiden mit einer Porosität entsprechend einem Volumen von 5 Vol % bis 85 Vol %, vorzugsweise 10 bis 50 Vol %, insbesondere 15 - 35 Vol %.

21. Mehrschichtiger Formkörper mit mehreren Schichten mit unterschiedlichen Eigenschaften in den Schichten erhältlich durch Aufbau des Formkörpers mittels Härtung einzelner Schichten und Auftrag neuer Schichten mit anderen Ausgangsmaterialien mit anderen physikalisch-technischen Eigenschaften.

22. Mehrschichtiger Formkörper mit mehreren Schichten mit unterschiedlichen Eigenschaften in den Schichten erhältlich durch Brennen einer homogenen Matrix in einem Gradientenofen.

23. Verfahren zur Herstellung eines Formkörpers nach Anspruch 21, wobei ein Aufbau des Formkörpers mittels Härtung einzelner Schichten und Auftrag neuer Schichten mit anderen Ausgangsmaterialien mit anderen physikalisch-technischen Eigenschaften erfolgt.

24. Verfahren zur Herstellung eines Formkörpers nach Anspruch 22, wobei eine homogene Matrix aus einem porösen anorganischen nichtmetallischen Material in einem Gradientenofen gebrannt wird.
